# EUROPEAN PATENT APPLICATION

(11) **EP 1 844 699 A2**
(43) Date of publication of application: **17.10.2007**
(21) Application number: 07007355.6
(22) Date of filing: 10.04.2007
(51) Int. Cl.: A61B 1/12

(54) **Apparatus for washing and disinfecting endoscope and brush unit for washing ducts of endoscope**

(30) Priority: 10.04.2006 JP 2006108112
(71) Applicant: OLYMPUS MEDICAL SYSTEMS CORP., Shibuya-ku Tokyo 151-0072 (JP)
(72) Inventor: Suzuki, Eiri, Tokyo 151-072 (JP); Hasegawa, Hitoshi, Tokyo 151-072 (JP); Suzuki, Shintaro, Tokyo 151-072 (JP); Noguchi, Toshiaki, Tokyo 151-072 (JP); Kobayashi, Kenichi, Tokyo 151-072 (JP); Ogawa, Akihisa, Tokyo 151-072 (JP)
(74) Representative: von Hellfeld, Axel

(57) **Abstract**

In an endoscope washing and disinfecting apparatus, a duct of the endoscope is washed by a washing brush having a brush chip and a shaft having a distal end to which the brush chip is detachably loaded. A brush-chip accommodating unit has a brush-chip accommodating chamber communicable to a flow passage of a nozzle detachably coupled to the duct. A shaft accommodating unit has a shaft accommodating chamber communicable to the brush-chip accommodating chamber, the shaft being selectively accommodated in the shaft accommodating chamber. A shaft driving unit drives the shaft to be fed to the duct from the shaft accommodating chamber and to be returned to the shaft accommodating chamber from the duct. In the brush-chip accommodating chamber, coupling and decupling means allow the brush chip to be coupled and decoupled with and from the shaft, during the shaft is driven to go back and forth.

## Description

### CROSS REFERENCE TO RELATED APPLICATION

The patent application related to and incorporates by reference Japanese Patent application No. 2006-108112 filed on April 10, 2006.

### BACKGROUND OF THE INVENTION

### [The Field of the Invention]

The present invention relates to an endoscope washing and disinfecting apparatus having a function for automatically inserting or retracting an elongated shaft and a brush chip, attached to a distal end of the shaft, through ducts of the endoscope accommodated in a washing and disinfecting bath for washing the ducts of the endoscope, and a brush unit

### [Related Art]

One means for inspecting and curing a body cavity is to use an endoscope. The endoscope includes an inserting section available to be inserted to the body cavity, which internally has various kinds of endoscope ducts (channels) such as a therapeutic-instrument insertion duct or the like that doubles as a gas-feed and water-feed duct, a forward-water-feed duct and a suction duct. Therefore, when used once, body fluid and feculence adhere onto the used endoscope in not only an external surface of the inserting section but also inside areas of the ducts of the endoscope. Therefore, after use, the external surface of the inserting section and the endoscope ducts need to be thoroughly washed and disinfected. To this end, in recent years, an endoscope washing and disinfecting apparatus has been put into practical use which can automatically wash and disinfect the endoscope even in its respective ducts.

Further, a piece of tissue or the like, removed from the body cavity in inspecting or making treatment with the use of the endoscope, passes across the ducts of the endoscope such as a therapeutic-instrument insertion duct doubling as a sucking duct. Thus, feculence easily adheres onto the duct and is hard to be removed merely in a washing process conducted with the endoscope washing and disinfecting apparatus.

Therefore, with a view to increasing washing capabilities of the various ducts of the endoscope, in general practice, a user inserts a washing brush, including a brush chip fixed to a distal end of, for instance, an elongated shaft, into the various ducts of the endoscope for rubbing and washing the same prior to conducting washing and disinfecting steps with the use of the endoscope washing and disinfecting apparatus. This allows the various ducts of the endoscope to be preliminarily washed, thereby removing feculence from the various ducts.

However, manually inserting the washing brush into the various ducts of the endoscope to preliminarily wash by rubbing and washing steps causes the user to bear troublesome work. This results in an increase in work time required for the endoscope to be washed and disinfected.

With the above view in mind, an attempt has been made to provide an endoscope washing and disinfecting apparatus as disclosed in Japanese Patent Application Publication No. 2003-10118. During the washing using such an apparatus, a washing brush is automatically inserted to various ducts of an endoscope and driven for back and forth drive movements. This makes it easy to wash the various ducts of the endoscope within a short period of time.

Further, another endoscope washing and disinfecting apparatus is disclosed in Japanese Patent Application Publication No. 8-275917. This apparatus includes a washing brush automatic inserter device. Therefore, automatically inserting a washing brush, corresponding to the washing brush, into various ducts of an endoscope enables the various ducts to be washed. In addition, the washing brush is detachably mounted on a device for driving the washing brush. This enables the washing brush, liable to wear, to be easily replaced.

Meanwhile, when inserting the washing brush into the various conducts of the endoscope for washing with the use of the endoscope washing and disinfecting apparatus, the washing brush, especially, a brush chip actually brought into contact with the ducts, is liable to wear. Such wearing induces a drop in a washing ability and the washing brush needs to be replaced.

In addition, the washing brush automatic inverter device for the endoscope, disclosed in Japanese Patent Application Publication No. 8-275917, has a structure in which the washing brush (cleaning brush) can be replaced. That is, such a structure enables both of a brush portion, corresponding to the brush chip, and a flexible shaft, corresponding to the shaft, to be replaced. However, the shaft is normally a component part that is expensive in cost. Therefore, if even the shaft is replaced each time a wear occurs on the brush chip, working hours on washing and disinfecting steps are prolonged, resulting in an increase in consumable supplies. Therefore, there has been a requirement for a device in which only a brush chip can be replaced.

### SUMMARY OF THE INVENTION

The present invention has been completed with the above view in mind and has an object to provide a endoscope washing and disinfecting apparatus and a brush unit which enable only a brush chip, inserted to ducts for back and force movements, to be easily replaced at low cost when automatically washing and disinfecting the ducts of an endoscope.

To achieve the above object, the present invention has one aspect to provide an endoscope washing and disinfecting apparatus for washing at least a duct of an endoscope. This apparatus a washing brush having a brush chip used for the washing and a shaft having a distal end to which the brush chip is detachably loaded; a nozzle having a flow passage and being detachably coupled to a connecting port of the duct of the endoscope; a brush-chip accommodating unit having a brush-chip accommodating chamber communicable to the flow passage; a shaft accommodating unit having a shaft accommodating chamber communicable to the brush-chip accommodating chamber, the shaft being selectively accommodated in the shaft accommodating chamber; and a shaft driving unit driving the shaft to be fed to the duct of the endoscope from the shaft accommodating chamber and to be returned to the shaft accommodating chamber from the duct of the endoscope. The shaft driving unit still comprises coupling means for coupling a shaft engaging member disposed at a distal end of the shaft to a brush-chip engaging member of the brush chip when the shaft is fed into the brush-chip accommodating chamber, and uncoupling means for uncoupling the shaft engaging member of the shaft from the brush-chip engaging member when the shaft is returned to the brush-chip accommodating chamber.

Further, to achieve the above object, another aspect of the present invention provides a brush unit including, among the above component parts, the washing brush, the nozzle, the brush-chip accommodating unit, the shaft accommodating unit, and the shaft driving unit, which are incorporated in a single unit and the single unit is detachably loaded to the apparatus.

Furthermore, to achieve the above object, a further aspect of the present invention provides a cartridge comprising a casing in which a brush-chip accommodating chamber is formed to removably accommodate therein a brush chip to detachably be loaded to a distal end of a shaft, the brush chip being driven go back and forth in and along a duct of an endoscope; and a flange portion formed in the brush-chip accommodating chamber and formed to allow the removable accommodation of the brush chip.

According to the present invention, the washing brush is detachably split into the brush chip and the shaft. Even when the cartridge accommodating only the brush chip is under use, the brush chip can be fed to and retracted from the duct of the endoscope through the back and forth movements of the shaft. Accordingly, only the brush chip may be suffice to be replaced, making it possible to easily replace only the brush chip at low cost.

### BRIEF DESCRIPTION OF THE DRAWINGS

In the accompanying drawings:
FIG. 1 is a perspective view showing an endoscope washing and disinfecting apparatus of a first embodiment according to the present invention with a top cover being opened;
FIG. 2 is a perspective view showing the endoscope washing and disinfecting apparatus with an endoscope, accommodated in an endoscope holder tray, being received in a washing and disinfecting bath under a state where the top cover is kept in a closed state;
FIG. 3 is a perspective view showing an outline of a structure of a brush unit;
FIG. 4 is a cross sectional view taken on line IV-IV of FIG. 3;
FIG. 5 is a front view showing a brush chip accommodating unit shown in FIG. 3;
FIG. 6 is perspective view showing parts of the brush chip and the shaft that form the washing brush shown in FIG. 4;
FIG. 7 is a cross sectional view showing the brush unit, together with parts of a washing and disinfecting bath and an endoscope manipulator section, in which a shaft engaging portion formed at a distal end of the shaft is coupled to a brush chip engaging portion of the brush chip;
FIG. 8 is an enlarged cross sectional view showing a coupling state between the shaft engaging portion and the brush engaging portion, shown in FIG. 7, in an enlarged scale;
FIG. 9 is a cross sectional view showing the brush unit, together with parts of the washing and disinfecting bath and the endoscope manipulator section, under a state where the washing brush, with the shaft and the brush chip kept in a coupled state, is fed out to a therapeutic-instrument duct;
FIG. 10 is a cross sectional view showing the brush unit, together with parts of the washing and disinfecting bath and the endoscope manipulator section, under a state where the washing brush is retracted from the therapeutic-instrument duct to a flow passage of a washing nozzle of the therapeutic-instrument duct;
FIG. 11 is a cross sectional view showing the brush unit, together with parts of the washing and disinfecting bath and the endoscope manipulator section, under a state where the shaft is uncoupled from the brush chip;
FIG. 12 is a perspective view showing an outline of a brush unit to be employed in an endoscope washing and disinfecting apparatus of a second embodiment according to the present invention;
FIG. 13 is a partial cross sectional view illustrating an internal structure of the brush unit of the second embodiment;
FIG. 14 is an illustrative view showing brush chips with large and small diameters and positions at which the brush chips are located on a cartridge; and
FIG. 15 is a flowchart showing an outline of a basic sequence of operations to be executed with the endoscope washing and disinfecting apparatus of the second embodiment.

### DETAILED DESCRIPTION OF THE PREFERRED EMBODIMENTS

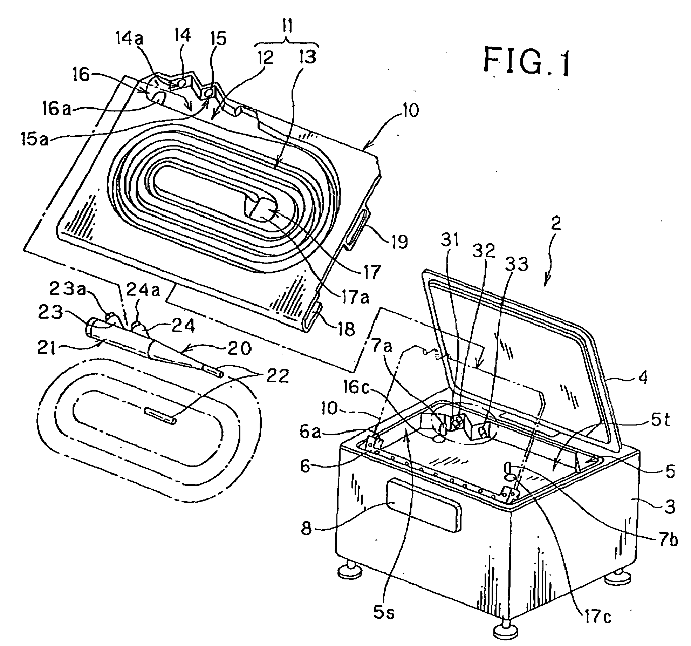

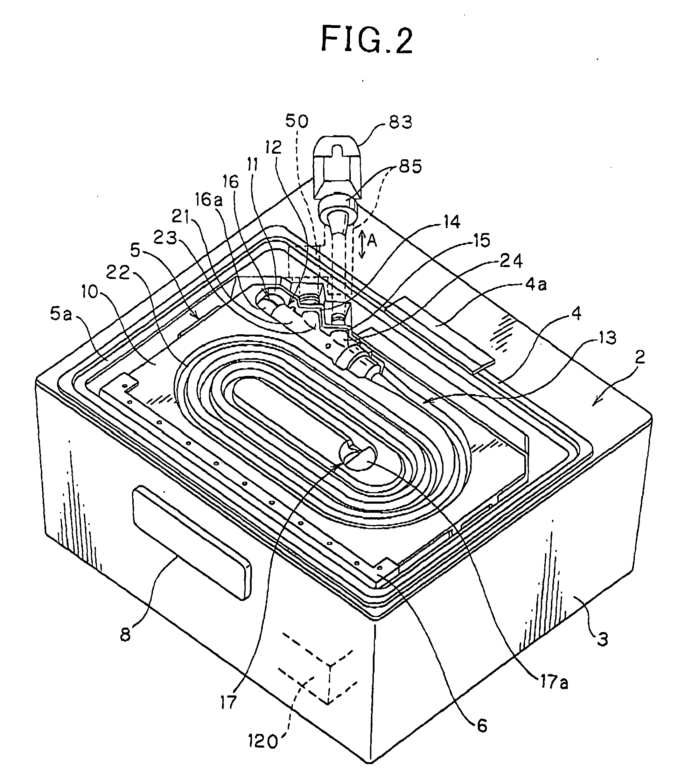

Now, an endoscope washing and disinfecting apparatus of a first embodiment according to the present invention will be described below with reference to FIGS. 1 to 11 of the accompanying drawings.
As shown in FIGS. 1 and 2, an endoscope washing and disinfecting apparatus 2 serves as a device for washing and disinfecting a used endoscope 20 and a related therapeutic-instrument or the like. This device 2 comprises a major part that includes a washing and disinfecting main body unit (hereinafter merely referred to as a "main body unit") 3 and a top cover 4 coupled to the main body unit 3 at an upper area thereof by means of, for instance, a hinge 4a for opening and closing capabilities.

Further, the upper area of the main body unit 3 is formed with a washing and disinfecting bath 5 with a given depth that has an upwardly opening endoscope reception opening adapted to be opened or closed with the top cover 4. In addition, the washing and disinfecting bath 5 has a structure available to accommodate therein an endoscope holder tray (hereinafter merely referred to as a "tray") 10 with the endoscope 20 carried thereon.

Further, a packing 5a (see FIG. 2) is disposed on the upper area of the main body unit 3 in an area, surrounding the washing and disinfecting bath 5, to provide a watertight sealing effect between the main body unit 3 and the top cover 4 when the top cover 4 is folded back to close the main body unit 3.

Furthermore, an operation panel 8 is mounted on the main body unit 3 at a front surface thereof in an area closer to, for instance, an operator. The operation panel 8 has various input operation switches such as, for instance, a washing and disinfecting start switch and a washing and disinfection mode selection switch or the like. In addition, the operation panel 8 provides a display of washing and disinfecting time and a display of an alarm or the like related to an operational failure. Moreover, the position of the operation panel 8 is not limited to the illustrated position provided that the operation panel 8 is provided on the main body unit 3.

The top cover 4 is formed of a hard and optically transparent resin member such as a so-called transparent resin member or half-transparent resin member. Accordingly, even under a circumstance where the endoscope reception opening of the washing and disinfecting bath 5 remains closed, a visual observation can be performed to check an inside of the washing and disinfecting bath 5 through the top cover 4.

A tray holder member 6 is mounted on the washing and disinfecting bath 5 of the main body unit 3 in a given position thereof, that is, in a position closer to an area near the operation panel 8 for the tray 10 to be mounted to or dismounted from the washing and disinfecting bath 5.

The tray holder member 6 takes a structure that is tiltable by means of, for instance, a pivot pin to tilt in an obliquely and upwardly oriented position for the tray 10 to be mounting or demounted and an accommodating position, parallel to a bottom surface 5t of the washing and disinfecting bath 5, to allow the tray 10 to be accommodated in the washing and disinfecting bath 5.

A first opening and closing protrusion 7a is located on a bottom surface 5t of the washing and disinfecting bath 5 in a given position thereof, that is, in a position in which, for instance, the hinge 4a is provided at an area remote from the operator. In addition, a water supply port 16c is located in an area close proximity to the first opening and closing protrusion 7a. Moreover, a second opening and closing protrusion 7b is located on the bottom surface 5t at a substantially central area thereof. In addition, a drain outlet 17c is located in an area near the second opening and closing protrusion 7b.

With the tray 10 accommodated in the washing and disinfecting bath 5, the first opening and closing protrusion 7a presses a lid member 16a of the tray 10 for the opening thereof and the second opening and closing protrusion 7b presses a lid member 17a of the tray 10 for the opening thereof.

The water supply port 16c serves to supply washing liquid, disinfecting liquid and flushing-out water or the like into the washing and disinfecting bath 5. The drain outlet 17c serves to drain washing liquid, disinfecting liquid and flushing-out water or the like out of the washing and disinfecting bath 5.

A fluid supply unit 50 and a brush unit 85 are provided on the washing and disinfecting bath 5 in an outer periphery thereof at an area, for instance, remote from the operator. In addition, a brush-unit cover 83, movable in an opening or closing position, is provided on an upper surface of the main body unit 3 so as to cover a part of the brush unit 85 in a way not to be exposed to the upper surface of the main body unit 3.

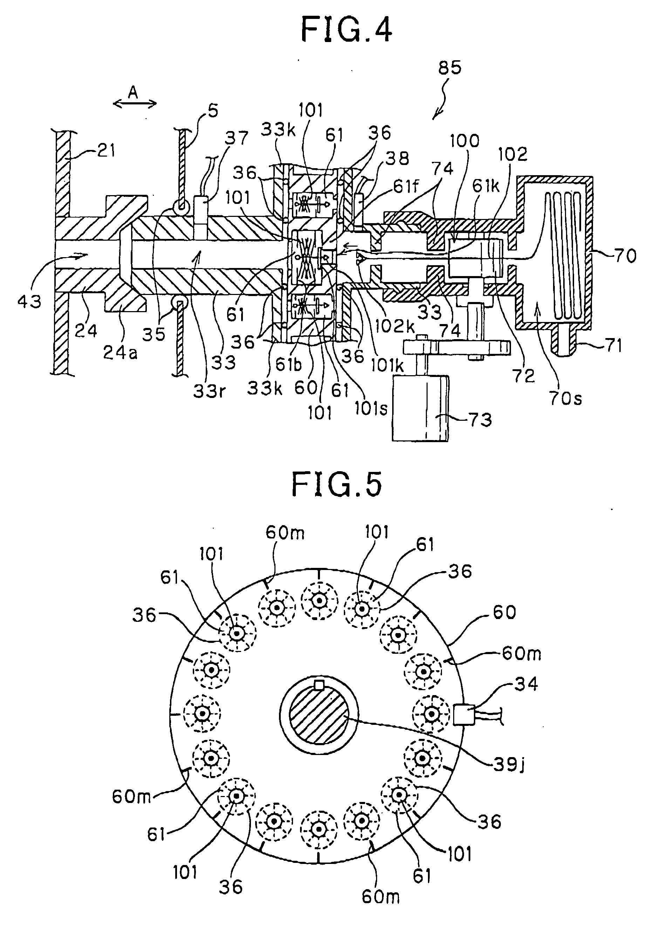

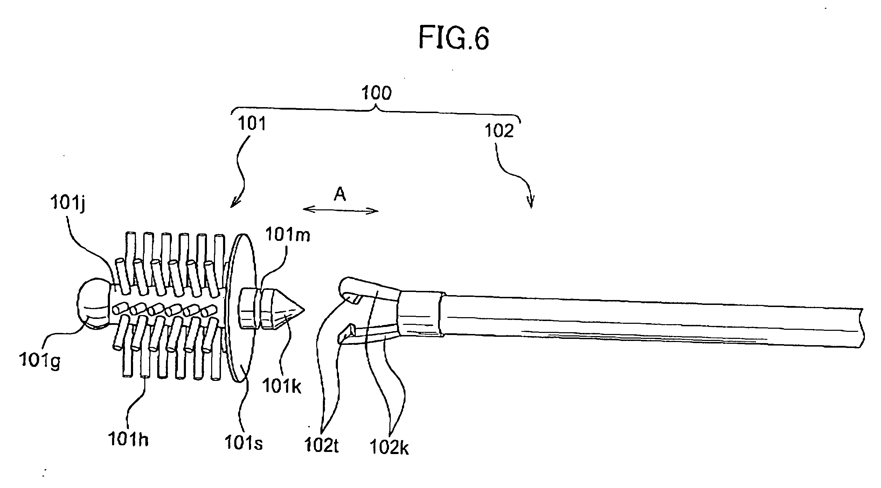

The fluid supply unit 50 serves to supply washing liquid and disinfecting liquid or the like to a gas-feed duct and a water-feed duct of the endoscope 20. The brush unit 85 serves to supply washing liquid and disinfecting liquid or the like to a therapeutic-instrument insertion duct 43 (see FIG. 4) of the endoscope 20 and insert a washing brush 100 (see FIG. 6) to the same in a manner as will be described below. Also, a detailed structure of the brush unit 85 will be described later In detail.

The fluid supply unit 50 has an area, facing the washing and disinfecting bath 5a, which is provided with a gas-feed-duct washing nozzle 31 and a water-feed-duct washing nozzle 32. The gas-feed-duct washing nozzle 31 and the water-feed-duct washing nozzle 32, actuated with a back and forth drive mechanism (not shown), move from a sidewall 5s of the washing and disinfecting bath 5 in a direction A perpendicular to the sidewall 5s (hereinafter referred to as a "back and forth traveling direction") to be remote from (in protruding) or closer to the sidewall 5s to be connectable to a gas-feed and water-feed fitting 23a of a duct coupling section 23 of the endoscope 20 in a manner described later.

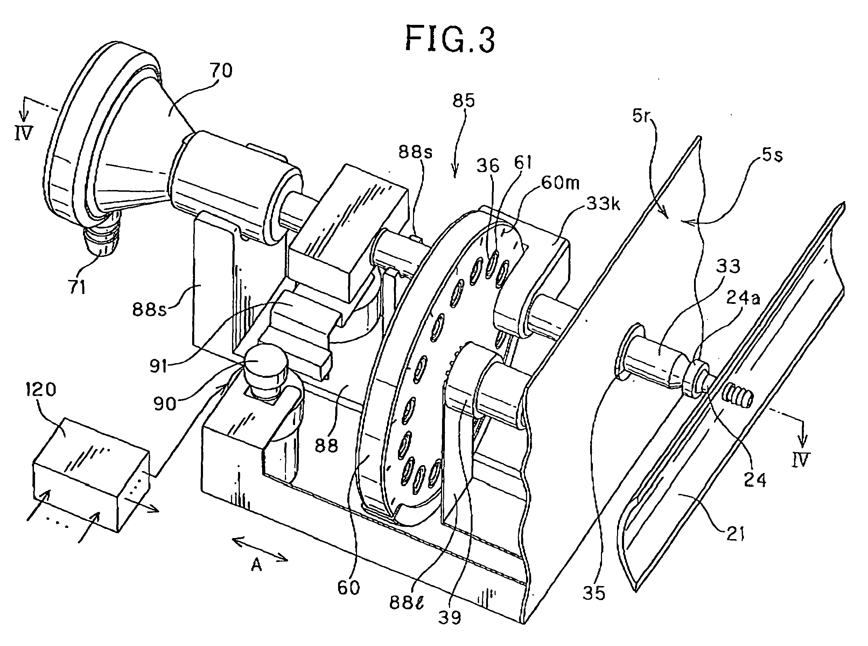

Further, the brush unit 85 is detachably mounted on the main body unit 3. The brush unit 85 has an area, exposed to the washing and disinfecting bath 5, which is formed with a therapeutic-instrument insertion-duct washing nozzle (hereinafter merely referred to as a "nozzle") 33 that plays a role as a duct connecting pipe. Back and forth moving mechanisms 90, 91 (see FIG. 3) move the nozzle 33 away from the sidewall 5s of the washing and disinfecting bath 5 (in protrusion) or toward the same in the back and forth traveling direction A to be connectable to a therapeutic-instrument insertion-duct fitting (hereinafter merely referred to as a "fitting") 24a of a therapeutic-instrument coupling section 24. In addition, a seal member 35 such as, for instance, an O-ring or the like, is fitted to between an outer periphery of a distal end of the nozzle 33 and an opening formed in the washing and disinfecting bath 5 as shown in FIG. 4.

The tray holder member 6, provided on the washing and disinfecting bath 5 of the main body unit 3, has holder portions 6a that detachably carry the tray 10 with the used endoscope 20 or the like being accommodated.

The endoscope 20, available to be detachably accommodated in the tray 10, comprises a major part that includes a manipulator section 21 and a flexible inserting section 22 contiguous with the manipulator section 21. In addition, the manipulator section 21 and the flexible inserting section 22 internally have a water-feed duct (not shown) for supplying water from an opening, placed in opposition to an objective lens, to a surface of the objective lens (not shown), located on a distal end of the inserting section 22, and a gas-feed duct (not shown) for feeding air or the like through the opening placed in opposition to the objective lens. Moreover, the manipulator section 21 and the flexible inserting section 22 may be internally provided with a forward-water-feed duct for feeding fluid such as water or the like from the distal end of the inserting section 22 to a forward area thereof.

Further, the manipulator section 21 and the inserting section 22 internally have the therapeutic-instrument insertion duct 43 (see FIG. 4), doubling as a sucking duct for sucking a piece of tissue or the like, which allows a therapeutic-instrument to protrude from the opening formed at the distal end of the inserting section 22.

The duct coupling section 23 and the therapeutic-instrument coupling section 24 are formed on the manipulator section 21 in, for instance, cylindrical shapes, respectively, so as to obliquely extending toward a base end thereof in a direction opposite to the longitudinally extending inserting section 22 of the manipulator section 21. In addition, the duct coupling section 23 and the therapeutic-instrument coupling section 24 are provided on the manipulator section 21 at positions spaced from each other along a longitudinal direction thereof.

A gas-feed and water-feed fitting 23a is formed on a distal end of the duct coupling section 23 so as to protrude therefrom and has respective openings exposed to the water-feed duct and the gas-feed duct of the manipulator 21. With the fluid supply unit 50 coupled to the duct coupling section 23, the gas-feed-duct washing nozzle 31 and the water-feed-duct washing nozzle 32 are connected to the gas-feed and water-feed fitting 23a

A therapeutic-instrument inserting fitting 24a is formed on a distal end of the therapeutic-instrument coupling section 24 so as to protrude therefrom and has an opening that extends through the therapeutic-instrument insertion duct 43 (see FIG. 4) and opened to the manipulator 21. With the brush unit 85 coupled to the therapeutic-instrument insertion duct 43, the nozzle 33 is connected to the fitting 24a.

In an alternative, further, none of the duct coupling section 23 and the therapeutic-instrument coupling section 24 may be provided on the manipulator 21 and the fittings 23a, 24a may be provided directly on the manipulator 21.

The upper surface of the tray 10 is formed with an accommodating concaved section 11 allowing the endoscope 20 to be accommodated for placement in a given position. The accommodating concaved section 11 is formed in a given profile with the account for external shapes and lengthwise dimensions, etc., of the manipulator section 21 and the inserting section 22 of the endoscope 20 to be accommodated. The accommodating concaved section 11 has a structure including a manipulator-accommodating section 12 for the manipulator section 21 to be located and an inserter-accommodating section 13 for the inserting section 22 to be located.

Accordingly, in using endoscopes 20 of a plurality of kinds with manipulator sections 21 and inserting sections 22 different in external shape and lengthwise dimension, a plurality of trays 10 may be selectively prepared to suit the endoscopes 20 of the respective kinds.

The manipulator-accommodating section 12 is formed with a duct receiver portion 14 and a therapeutic-instrument receiver portion 15 for accommodating the duct coupling section 23 and the therapeutic-instrument coupling section 24 of the endoscope 20 accommodated in the accommodating concaved portion 11.

The duct receiver portion 14 is formed with an opening 14a to which distal ends, extending in the back and forth traveling direction A, of the gas-feed-duct washing nozzle 31 and the water-feed-duct washing nozzle 32 are inserted. The therapeutic-instrument receiver portion 15 is formed with an opening 15a to which a distal end, extending in the back and forth traveling direction A, of the nozzle 33 is inserted.

A first water supply and drainage port 16 is formed on the manipulator-accommodating section 12 at a bottom wall thereof in a given position for supplying washing water and disinfecting water or the like thereto and draining the same therefrom. With the endoscope 20 accommodated in the accommodating concaved section 11, further, the first water supply and drainage port 16 is located in an area close proximity to a base end portion of the manipulator section 21 of the endoscope 20 and assumes a position close proximity to the water supply port 16c when the tray 10 is accommodated in the washing and disinfecting bath 5.

Further, a second water supply and drainage port 17 is formed on the inserter-accommodating section 13 at the bottom wall in another given position thereof for supplying washing water and disinfecting water or the like thereto and draining the same therefrom. In addition, with the endoscope 20 accommodated in the accommodating concaved section 11, the second water supply and drainage port 17 is located in an area close proximity to a distal end of the inserting section 22 of the endoscope 20 and assumes a position In the vicinity of a water drain outlet 17c when the tray 10 is accommodated in the washing and disinfecting bath 5.

Furthermore, the first and second water supply and drainage ports 16, 17 have lid members 16a, 17a mounted for opening and closing capabilities, respectively. The lid members 16a, 17a take the form of structures that are normally kept under closed conditions, respectively, with own weights or the own weights and additional biasing forces exerted by biasing means (not shown).

Consequently, when the used endoscope 20 is accommodated in the accommodating concaved section 11, no contaminant or body fluid, adhered onto the endoscope 20, is leaked from the first and second water supply and drainage ports 16, 17. Therefore, the endoscope 20 can be hygienically conveyed with the endoscope 20 accommodated in the accommodating concaved section 11 of the tray 10.

A mounting portion 18 is formed on the tray 10 on one side thereof extending in a direction perpendicular to a longitudinal direction in FIG. 2. When the tray 10 is accommodated In the washing and disinfecting bath 5 of the main body unit 3, the mounting portion 18 is fitted to the holder portions 6a of the tray holder member 6, provided on the washing and disinfecting bath 5, and has a shape formed in, for instance, a U-shaped configuration in conformity to internal profiles of the holder portions 6a.

The tray 10 has carrier grips 19 formed on both sides facing the longitudinal direction in FIG. 2. The carrier grips 19, available to be gripped when transporting the tray 10 receiving the endoscope 20, are formed so as to protrude downward from the tray 10. Therefore, no interference takes place between the carrier grips 19 and the top cover 4 when the tray 10 is accommodated in the washing and disinfecting bath 5.

Now, the structure of the brush unit 85 will be described below with reference to FIGS. 3 to 6.

As set forth above, the brush unit 85 is detachably mounted to and dismounted from the main body unit 3 of the washing and disinfecting apparatus 2. As shown in FIGS. 3 and 4, the brush unit 85 includes major component parts such as the nozzle 33 formed in an elongated tubular shape, a brush chip cartridge 60, disposed in a halfway of the nozzle 33 to serve as a brush chip accommodating unit, and a shaft cassette 70 serving as a shaft receiving unit.

The nozzle 33 internally has a flow passage 33r through which a washing brush 100, described below, and washing liquid or the like pass. With the nozzle 33 coupled to the fitting 24a of the therapeutic-instrument coupling section 24, the flow passage 33r is brought into fluid communication with the therapeutic-instrument insertion duct 43 internally formed in the endoscope 20. This allows the washing brush 100 and fluid such as washing liquid or the like to be supplied to the therapeutic-instrument insertion duct 43.

As shown in FIGS. 3 and 4, further, the nozzle 33 has the distal end whose outer periphery is fitted to the opening formed in the washing and disinfecting bath 5 via the seal member 35 such as the O-ring or the like for sliding engagement.

Furthermore, a rear half portion of the nozzle 33 is supported with a column support 88s standing upright from a base 88 as shown in FIG. 3. In addition, the nozzle 33 has a midway formed in a crank portion 33k in a "U"-shape on a plane surface as shown in FIG. 3. The crank portion 33k has a clearance in which the disc-like brush chip cartridge 60, having a given thickness along the back and forth traveling direction A, is disposed in an overlapping relationship with the crank portion 33k on the plane surface as shown in FIG. 3.

As shown in FIGS. 3 and 5, the brush chip cartridge 60 is rotatably supported with a motor shaft 39j of a unit motor 39. The motor unit 39 is a rotation control unit that is supported on an "L"-shaped column support 881 extending from a rear surface 5r of the sidewall 5s of the washing and disinfecting bath 5 and standing upright in an "L"-shape configuration. The brush chip cartridge 60 includes a suitable member such as, for instance, a disposable member that is detachably mounted on the motor shaft 39j.

Moreover, the brush chip cartridge 60 is formed with a plurality of brush-chip accommodating chambers (hereinafter merely referred to as "accommodating chambers") 61 that are, for instance, circular on a plane surface as shown in FIGS. 3 and 5. The plural accommodating chambers 61 are formed on the brush chip cartridge 60 at circumferentially spaced positions with a given distance and extend through the brush chip cartridge 60 in the back and forth traveling direction A. Therefore, the brush chip cartridge 60 takes the form of a so-called revolver structure in which the plural accommodating chambers 61 rotate about the center of the motor shaft 39j.

While the brush chip cartridge 60 has been shown in FIG. 5 with reference to a structure formed with 16 accommodating chambers 61, the present invention is not limited to the number of such 16 pieces. In addition, it is needless to say that the planar shape of each accommodating chamber 61 is not limited to the circular configuration.

Seal members 36 such as, for instance, O-rings or the like are disposed in areas near outer peripheral edges of communicating ports of the respective accommodating chambers 61 formed on front and rear end surfaces of the brush chip cartridge 60, respectively, in the back and forth traveling direction A to be brought into abutting engagement with the crank portion 33k as shown in FIG. 5.

As shown in FIG. 5, further, positioning indexes 60m are formed on the brush chip cartridge 60 at the front end face thereof, facing the back and forth traveling direction A, in areas close proximity to the communication ports of the plural accommodating chambers 61, respectively. The positioning indexes 60m play a role as indexes that are detected using a rotation-positioning sensor 34, described later, in, for instance, magnetic or optical phases.

The accommodating chambers 61 have the communicating ports formed in fluid communication with front and rear end sides facing the back and forth traveling direction A. As shown in FIG. 4, further, the communicating ports 61k closer to rear end portions are formed with radially inward flange portions 61f protruding toward insides of the chambers.

Furthermore, radially inward flange portions 61b, made of, for instance, a resilient member with a thin wall thickness in the back and forth traveling direction A, are formed in the accommodating chambers 61 in areas close proximity to the radially inward flange portions 61f and front end areas in the back and forth traveling direction A.

With the brush chip cartridge 60 being rotated, among the plural accommodating chambers 61, either one of the accommodating chambers 61 is positioned such that a portion of the brush chip cartridge 60 overlaps the clearance of the crank portion 33k on the plane surface. In this moment, one accommodating chamber 61 is brought into fluid communication with the flow passage 33r of the nozzle 33 and a shaft accommodating chamber (hereinafter merely referred to as a "accommodating chamber") 70s, described later, of the shaft cassette 70.

A control device 120 allows the either one accommodating chamber 61, the flow passage 33r of the nozzle 33 and the shaft accommodating chamber 70s to be automatically brought into fluid communication with each other using the positioning indexes 60m. That is, the rotation-positioning sensor 34 of the motor unit 39, disposed in an area near an outer periphery of the brush chip cartridge 60 at a front end thereof, detects the positioning index 60m provided for each accommodating chamber 61. Such detected information is delivered to the control device 120. The control device 120 controls the rotation of the motor unit 39 for thereby controlling a rotary state of the brush chip cartridge 60. This provides the fluid communication mentioned above.

The control device 120 includes a computer that is incorporated in the main body unit 3 to perform operations according to programs describing a basic sequence of operations that is preliminarily set. To this end, the control device 120 has memories storing therein programs and a CPU (Central Processing Unit) for performing computations in accordance with the stored programs.

Further, upon receipt of a brush chip 101 (a brush portion except for a shaft of the washing brush 100) in either one accommodating chamber 61, the motor unit 39, described below, rotates the brush chip cartridge 60 using the positioning indexes 60m and the rotary positioning sensor 34 so as to allow the accommodating chamber 61 adjacent to the accommodating chamber 61 on the plane surface which accommodates the brush chip to be brought into fluid communication with the flow passage 33r of the nozzle 33 and the accommodating chamber 70s.

As shown in FIG. 4, the brush chip 101 of the washing brush 100 is accommodated in the accommodating chamber 61. The brush chip 101 has a core member 101j and a brush portions 101h wound thereon for rubbing and washing the therapeutic-instrument insertion duct 43. As shown in FIGS. 4 and 6, the core member 101j has a distal end, facing the back and forth traveling direction A, on which a sphere-like protecting member 101g is mounted for preventing the therapeutic-instrument insertion duct 43 from being damaged. In addition, it doesn't matter if the protecting member 101g is formed integrally with the core member 101j.

As shown in FIGS. 4 and 6, further, the core member 101j of the brush chip 101 has a rear end, facing the back and forth traveling direction A, which is formed with a cone-shaped brush engaging portion (hereinafter merely referred to as an "engaging portion") 101k. The engaging portion 101k is made of a resilient member to be detachably coupled to a shaft engaging portion 102k formed on the shaft 102 at a distal end thereof, which will be described later. Also, it doesn't matter if the engaging portion 101k is integrally formed with the core member 101j.

As shown in FIGS. 4 and 6, furthermore, a stopper member 101s is mounted on the core member 101j in a position between the brush portion 101h and the engaging portion 101k. The stopper member 101s is comprised of a disc-like member that is wider on the plane surface than the communicating port 61k. In addition, it doesn't matter if the stopper member 101s is integrally formed with the core member 101s.

The stopper member 101s is brought into abutting contact with the radially inward flange portion 61f. This prevents the brush chip 101 from getting out of the accommodating chamber 61 into the accommodating chamber 70s via the communicating port 61k at the rear end. In particular, the stopper member 101s prevents the brush chip 101 from being accommodated in the cassette 70 to which a base end of the nozzle 33 is connected.

As shown in FIGS. 3 and 4, moreover, the shaft cassette 70, supported on the columns support 88s of the base 88, is detachably connected to the nozzle 33 a rear end thereof in the back and forth traveling direction A. In addition, although the shaft cassette 70 is made of, for instance, a disposable member, the shaft cassette 70 may not be disposable.

As shown in FIGS. 4 and 6, an elongated shaft 102 of the washing brush 100 is wound for placement in the accommodating chamber 70s of the shaft cassette 70. Also, the shaft 102 has a distal end carrying thereon the shaft engaging portion (hereinafter merely referred to as "engaging portion") 102k that includes claw portions 102t detachably coupled to an engaging surface 101m of the cone-shaped engaging portion 101k of the brush chip 101. Also, it doesn't matter if the engaging portion 102k is integrally formed with the shaft 102.

Further, the accommodating chamber 70s is available to freely communicate with either one accommodating chamber 61 of the brush cartridge 60.

Furthermore, the shaft cassette 70 has an outer periphery formed with a washing and disinfecting liquid supply port 71 in communication with the accommodating chamber 70s. With the washing and disinfecting liquid supply port 71 connected to a washing liquid supply source or a disinfecting liquid supply source or the like which are not shown, the accommodating chamber 70s is supplied with washing liquid or disinfecting liquid or the like.

The shaft 102, accommodated in the accommodating chamber 70s of the shaft cassette 7, is fed to the therapeutic-instrument insertion duct 43 of the endoscope 20 through either one accommodating chamber 61 in fluid communication with the flow passage 33r of the nozzle 33. At the same time, the brush feeding-returning roller 72, playing a role as a shaft feeding and returning unit for feeding the delivered shaft back to the accommodating chamber 70s, and the brush feeding-returning motor 73, playing a role as the shaft feeding and returning unit, are connected to each other.

Further, the brush feeding-returning roller 72 and the brush feeding-returning motor 73 feed the shaft 102 to either one accommodating chamber 61 in relevant fluid communication. This allows the engaging portion 102k of the shaft 102 to be coupled to the engaging portion 101k of the brush chip 101, accommodated in the accommodating chamber 61, or the fed out shaft 102 to be returned. This causes the engaging portion 102k, held in engagement, to be uncoupled from the engaging portion 101k of the brush chip 101.

The shaft cassette 70 is internally formed with a brush guide 74 having a radially inward flange shape formed with a communicating bore. The brush guide 74 allows the communication bore to guide such that when the shaft 102 is fed from the reception chamber 101, the engaging portion 102k of the shaft 102 is brought Into engagement with the engaging portion 101k of the brush chip 101 accommodated in the accommodating chamber 61.

Further, a traveling position of the delivered shaft 102, that is, a traveling position of the brush chip 101 is controlled with the control device 120. That is, as shown in FIG. 4, the traveling position of the delivered shaft 102 is detected with a leading-end position detecting sensor 37, located in the nozzle 33 at a distal end thereof in the back and forth traveling direction A, and a trailing-end position detecting sensor 38 located in the nozzle 33 at a rear end thereof in the back and forth traveling direction A. Thus, the traveling position of the shaft 102 is controlled by the control device 120 based on the detected information.

More particularly, the leading-end position detecting sensor 37 outputs positioning information related to a retracted limit position of the brush chip 101 in the back and forth traveling direction A when the washing brush 100 is inserted to the therapeutic-instrument insertion duct 43 and driven to move back and forth therein for rubbing and washing the same. This positioning information is applied to the control device 120, which in turn executes such positioning control.

Further, the trailing-end position detecting sensor 38 outputs positioning information related to a back-feed limit position of the brush chip 101 in the back and forth traveling direction A when the shaft 102 is fed back to a position at which the engaging portion 102k is uncoupled from the engaging portion 101k. This positioning information is applied to the control device 120, which in turn executes such positioning control. This prevents the engaging portion 102k from entering the accommodating chamber 70s of the shaft cassette 70.

As shown in FIG. 3, the base 88 carries thereon a back and forth drive motor 90 and a guide member 91, which form a back and forth drive mechanism for moving the nozzle 33 back and forth in the back and forth traveling direction A.

The guide member 91 includes a rectangular column fixedly secured to a bottom wall of the base 88 in an area near the base end of the nozzle 33 with respect to the back and forth traveling direction A and extends in the back and forth traveling direction A. With a gear of the back and forth drive motor 90 held in meshing engagement with a side wall of the guide member 91, the guide member 91 causes the nozzle 33, fixed to the guide member 91, to move back and forth in the back and forth traveling direction A.

That is, the back and forth drive motor 90 and the guide member 91 allow the nozzle 33 to be coupled to and uncoupled from the fitting 24a of the therapeutic-instrument coupling section 24 of the endoscope 20. In addition, the back and forth drive motor 90 and the guide member 91 may allow the gas-feed-duct washing nozzle 31 and the water-feed duct washing nozzle 32 of the fluid supply unit 50 (see FIG. 2) to move in the back and forth traveling direction A such that the respective nozzles 31, 32 are coupled to or uncoupled from the gas-feed and water-feed fitting 23a of the duct coupling section 23 of the endoscope 20.

Next, the operation and advantages of the endoscope washing and disinfecting apparatus 2 with such a structure will be described below with reference to FIGS. 1 to 6 and FIGS. 7 to 11.

First, a used endoscope 20 is accommodated and placed in the accommodating concaved section 11 of the tray 10. In particular, the inserting section 22 is accommodated and placed in the inserter-accommodating section 13. The distal end of the duct coupling section 23 of the manipulator 21 is inserted to the opening 14a of the duct receiver portion 14. Then, the distal end of the therapeutic-Instrument coupling section 24 is inserted to the opening 15a of the therapeutic-instrument receiver portion 15. As a result, the duct coupling section 23 and the therapeutic-instrument coupling section 24 are placed and positioned in a given position of the manipulator-accommodating section 12.

After the endoscope 20 has been accommodated in the accommodating concaved section 11, the tray 10 is coupled to the tray holder member 6 located in the mounting and dismounting position as shown by a phantom line in FIG. 1. In this case, the mounting section 18 of the tray 10 is fitted to the holder portions 6a of the tray holder member 6. Thereafter, the tray holder member 6 is manually or automatically rotated from the mounting and demounting position into an accommodating position of the washing and disinfecting bath 5. This allows the tray 10, located in the tray holder member 6, to be accommodated at the accommodating position in the washing and disinfecting bath 5 with the rotating movement of the tray holder member 6 as shown in FIG. 2.

In this moment, the tray 10, accommodated in the washing and disinfecting bath 5, causes the position of the duct coupling section 23 to be defined so as to face in opposition to the fluid supply unit 50 and the position of the therapeutic-instrument coupling section 24 is defined to face in opposition to the brush unit 85.

Subsequently, the first opening and closing protrusion 7a, protruding from the bottom wall 5t of the washing and disinfecting bath 5, pushes up the lid member 16a, thereby opening the first water supply and drainage port 16. At the same time, the second opening and closing protrusion 7b pushes up the lid member 17a, thereby opening the second water supply and drainage port 17.

Thereafter, the top cover 4 is manually or automatically moved in a closing direction and the endoscope accommodating opening of the washing and disinfecting bath 5 is closed as shown in FIG. 2. In this moment, further, the packing 5a, provided on the main body unit 3 in the upper surface thereof, provides a watertight sealing effect between the top cover 4 and the main body unit 3. Accordingly, during the washing and disinfecting operation, no liquid flushes out of the washing and disinfecting bath 5 to an area outside the main body unit 3.

Then, in a case where, for instance, the back and forth drive motor 90 and the guide member 91 combine a back and forth drive mechanism of the fluid supply unit 50 and a back and forth drive mechanism of the brush unit 85, the back and forth drive motor 90 and the guide member 91 move the nozzle 33 toward a leading end position in the back and forth traveling direction A. This causes the nozzle 33 to be coupled to the fitting 24a of the therapeutic-instrument coupling section 24 of the endoscope 20.

At the same time, the back and forth drive motor 90 and the guide member 91 move the gas-feed-duct washing nozzle 31 and the water-feed duct washing nozzle 32 toward the leading end position in the back and forth traveling direction A. This causes the gas-feed-duct washing nozzle 31 and the water-feed duct washing nozzle 32 to be coupled to the gas-feed and water-feed fitting 23a of the duct coupling section 23 of the endoscope 20.

Next, the motor unit 39 is driven to rotate the brush chip cartridge 60. Then, the rotation-positioning sensor 34 detects the index 60m to output detected information indicative of the index 60m. Upon receipt of detected information, the control device 120 allows either one of the plural accommodating chambers 61, formed in the brush chip cartridge 60, to be brought into fluid communication with the flow passage 33r of the nozzle 33 and the accommodating chamber 70s.

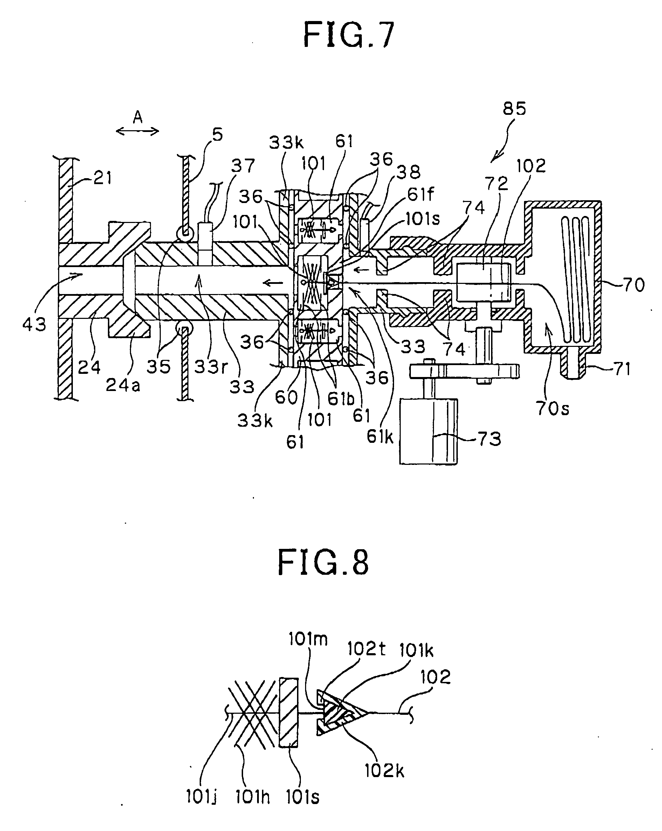

Thereafter, with the brush feeding-returning roller 72 and the brush feeding-returning motor 73 being driven, the shaft 102 of the washing brush 100 is fed out of the accommodating chamber 70s of the shaft cassette 70. Further, the brush guide 74 guides the shaft 102 such that the shaft engaging portion 102k, formed at the distal end of the shaft 102, is coupled to the engaging portion 101k of the brush chip 101 accommodated in either one accommodating chamber 61 as shown in FIG. 7. That is, the shaft 102 engages the brush chip 101.

When this takes place, as shown in FIG. 8, the two claw portions 102t of the engaging portion 102k engage the engaging surface 101m of the cone-shaped engaging portion 101k in a clamping effect. This allows the shaft engaging portion 102k to engage the engaging portion 101k.

Further, during the engaging movement between the engaging portion 102k and the engaging portion 101k, a tendency occurs for the shaft 102 to push the brush chip 101 out of the communicating port formed at the leading end area of the accommodating chamber 61. However, the brush portion 101h of the brush chip 101 is brought into abutting contact with the radially inward flange portion 61b formed in the accommodating chamber 61 at the distal end thereof. This prevents the brush chip 101 from moving out of the communicating port, formed at the distal end of the accommodating chamber 61, before the engagement takes place.

Upon engagement of the engaging portion 102k with the engaging portion 101k, the brush feeding-returning roller 72 and the brush feeding-returning motor 73 are further driven. This causes the washing brush 100, with the brush chip 101 coupled to the shaft 102, to be fed out of the fitting 24a and inserted to the therapeutic-instrument insertion duct 43.

Thereafter, the brush feeding-returning roller 72 and the brush feeding-returning motor 73 drive the shaft 102 back and forth. This allows the brush portion 101h of the brush chip 101, forming the inserted brush 100, to rub and wash the therapeutic-instrument insertion duct 43. That is, the therapeutic-instrument insertion duct 43 is washed.

In addition, the washing brush 100 is inserted to the therapeutic-instrument insertion duct 43 until the brush 100 protrudes out of the opening of the therapeutic-instrument insertion duct 43 placed at the distal end of the inserting section 22 of the endoscope 20.

Further, during such washing operation, the washing and disinfecting liquid supply port 71 is connected to the washing and disinfecting liquid supply source. Thus, washing liquid is supplied to the therapeutic-instrument insertion duct 43 via the accommodating chamber 70s, the brush accommodating chamber 61, the flow passage 33r and the fitting 24a. In addition, supplied washing liquid flows through the opening of the therapeutic-instrument insertion duct 43 provided at the distal end of the inserting section 22 to the second water supply and drain port 17 of the tray 10 and drains from the drain outlet 17c of the washing and disinfecting bath 5.

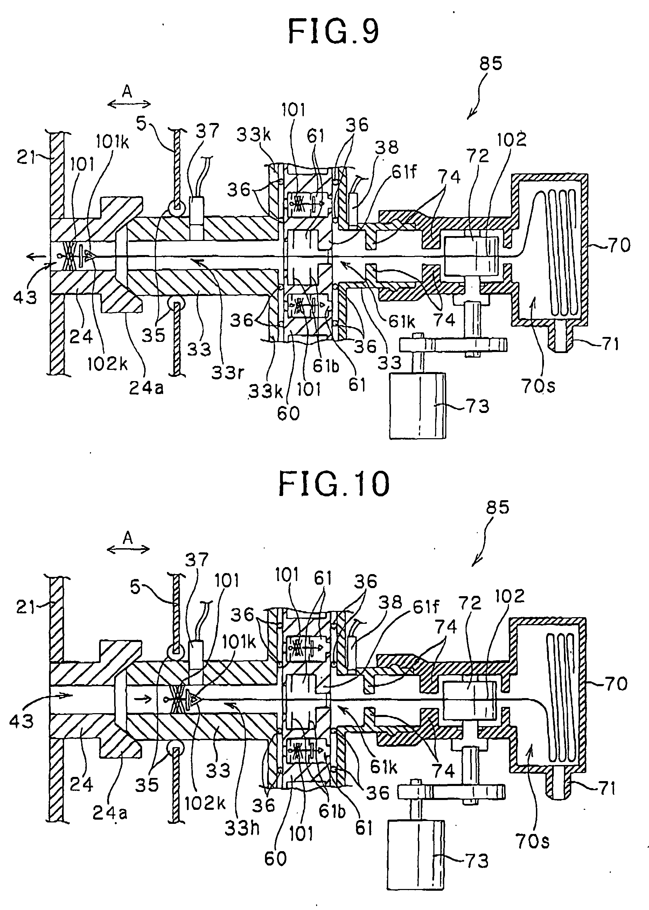

Furthermore, the distal-end-position detecting sensor 37 is provided on the nozzle 33 at the distal end thereof. Therefore, during the washing operation, the brush unit 85 operates such that, as shown in FIG. 10, no brush chip 101 is retracted through the flow passage 33r to an area rearward of the distal-end-position detecting sensor 37 in the back and forth traveling direction A.

Moreover, in a substantially synchronism with the washing of the therapeutic-instrument insertion duct 43, the nozzles 31, 32 supply washing liquid to the water-feed duct and the gas-feed duct of the endoscope 20 via the fitting 23a for washing the respective ducts. Even in such a case, supplied washing liquid flows out of the respective openings of the water-feed duct and the gas-feed duct provided at the distal end of the inserting section 22 to drain from the drain outlet 17c of the washing and disinfecting bath 5 via the second water supply and drain port 17 of the tray 10.

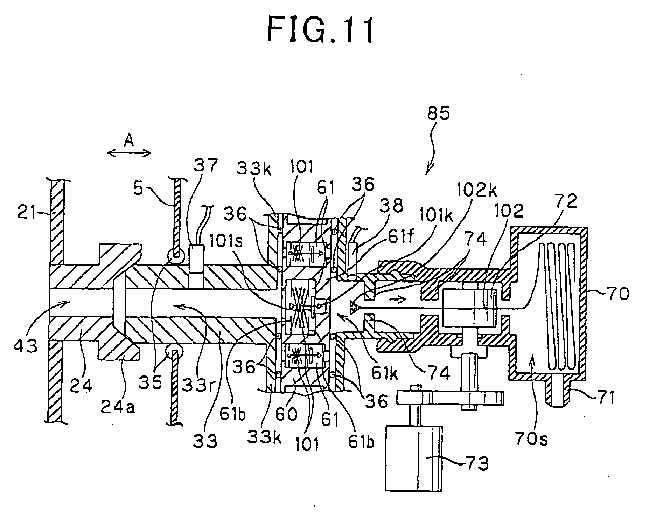

After the washing, the brush feeding-returning roller 72 and the brush feeding-returning motor 73 draw the shaft 102 back to the accommodating chamber 70s of the shaft cassette 70. This causes the brush chip 101 to get out of the therapeutic-instrument insertion duct 43 to be retracted back to the flow passage 33r as shown in FIG. 10. Then, as shown in FIG. 11, the brush chip 101 is accommodated in the accommodating chamber 61 of the brush chip cartridge 60. In this moment, the brush chip 101 is accommodated in the same accommodating chamber 61 as that from which the brush chip 101 is drawn in a preceding stage.

When this takes place, the brush feeding-returning roller 72 and the brush feeding-returning motor 73 are further continuously driven. Therefore, even if the shaft 102 is continuously drawn back, the stopper 103 of the brush chip 101 is brought into abutting contact with the radially inward flange portion 61f of the accommodating chamber 61. Thus, no probability occurs for the brush chip 101 to fly out of the communicating port 61k of the accommodating chamber 61 at the rear side thereof to the accommodating chamber 70s.

Further, the shaft 102 is continuously drawn back. This causes the engaging portion 102k to be uncoupled from the engaging portion 101k at timing when the stopper 103 of the brush chip 101 is brought into abutting contact with the radially inward flange portion 61f.

Upon disengagement, the engaging portion 102k is retracted to the accommodating chamber 70s. However, the shaft 102 is controlled in response to detected information output from the rearward-position detecting sensor 38. Therefore, no probability takes place for the engaging portion 102k from being retracted to the rear side in the back and forth traveling direction A. That is, no happening takes place for the uncoupled engaging portion 102k to be accommodated in the accommodating chamber 70s.

Upon receipt of the brush chip 101 in the accommodating chamber 61, the motor unit 39 is operated to rotate the brush cartridge 60 under which the rotation-positioning sensor 34 detects the index 60m. Therefore, the accommodating chamber 61, placed adjacent to the accommodating chamber 61 on the plane surface to which the brush chip 101 is accommodated, is brought into fluid communication with the flow passage 33r of the nozzle 33 and the accommodating chamber 70s.

Further, in a case where the brush chip cartridge 60 has sixteen accommodating chambers 61, the engagement between the engaging portion 102k and the engaging portion 101k, the insertion of the washing brush 100 into the therapeutic-instrument insertion duct 43, the disengagement of the engaging portion 102k from the engaging portion101k and the rotating movement of the brush chip cartridge 60 are repeatedly carried out 16 times. That is, with the use of one brush chip cartridge 60, the respective therapeutic-instrument insertion ducts 43 of sixteen endoscopes 20 can be rubbed and washed with brush chips 101 of new washing brushes 100 every time.

After all of the sixteen brush chips 101, accommodated in the sixteen accommodating chambers 61, respectively, have been completely used, the brush chip cartridge 60 is removed from the brush unit 85, that is, the brush chip cartridge 60 is removed from the motor shaft 39j for disposal.

In addition, a judgment on whether or not all of the sixteen brush chips 101 are completely used may suffice to be made by finding whether or not the rotation positioning sensor 34 detects the rotary positions sixteen times. The control device 120 makes a judgment based on detecting information whether or not all of the brush chips 101 are spent.

Upon detecting the rotary positions sixteen times, moreover, a user may be provided with an alarm sound or the like to give the awareness for replacement of the brush chip cartridge 60. In addition, it doesn't matter if the sixteen brush chips 101 and brush accommodating chambers 61 are used for washing the therapeutic-instrument insertion ducts 43 again after the washing step.

Thus, with the present embodiment, the brush chip cartridge 60, having the plural accommodating chambers 61 for accommodating the brush chips 101, respectively, is rotated like a revolver. Then, either one accommodating chamber 61 is brought into fluid communication with the flow passage 33r of the nozzle 33 and the accommodating chamber 70s of the shaft cassette 70 in which the shaft 102 is accommodated. The engaging portion 102k, formed on the distal end of the shaft 102 fed from or fed back to the accommodating chamber 70s, is detachably engageable with the engaging portion 101k of the brush chip 101 accommodated in the accommodating chamber 61 in fluid communication.

Accordingly, no need arises for an expensive shaft 102 to be replaced, making it possible to replace only the brush chips 101.

With the present embodiment, further, the brush chip cartridge 60 has another advantage to be detachably coupled to the nozzle 33. That is, after all of the brush chips 101 accommodated in the plural accommodating chambers 61 of the brush chip cartridge 60, respectively, have been completely used, merely replacing the brush chip cartridge 60 enables the used brush chips 101 to be disposed in an easy way. In addition, new brush chips 101 can be set to the brush unit 85.

With the present embodiment, furthermore, the stopper member 101s are provided on the brush chips 101 at the rear ends thereof with respect the back and forth traveling direction A. Therefore, during the operations of the brush feed roller 72 and the motor 73 drawing the shaft 102 back to the accommodating chamber 70s, the stopper member 101s is brought into abutting contact with the radially inward flange portion 61f in the accommodating chamber 61. Thereafter, the engaging portion 102k is uncoupled from the engaging portion 101k. Thus, no happening takes place for the brush chip 101 to fly out of the rear communicating port 61k to the accommodating chamber 70s. This enables the brush chip 101 to be reliably accommodated in the accommodating chamber 61.

With the present embodiment, moreover, the shaft cassette 70 is detachably coupled to the nozzle 33. Therefore, if the shaft 102 is worn, the rearward-position detecting sensor 38 is turned off and the shaft 102 and the engaging portion 102k are completely accommodated in the accommodating chamber 70s, after which the shaft cassette 70 is replaced. Performing only such operation enables the worn shaft 102 to be disposed, thereby making it possible to set a new shaft 102 to the brush unit 85.

### (Modified Forms)

Also, the present invention is not limited to the structure of the first embodiment set forth above and can be implemented in various modified forms. Hereunder, examples of these modifications will be described.

### (First Modified Form)

With the first embodiment set forth above, the brush unit 85 is structured such that the washing brush 100 is inserted to the therapeutic-instrument insertion ducts 43 of the endoscope 20 for use in washing the therapeutic-instrument insertion ducts 43. However, the washing brush 100 may be used, but not limited, for insertion to the water-feed duct or the gas-feed duct of the endoscope 20 for washing the water-feed duct or the gas-feed duct.

### (Second Modified Form)

With the first embodiment set forth above, further, the endoscope 20 has a structure so as to be accommodated in the washing and disinfecting bath 5 under a received state in the tray 10. However, the endoscope 20 may have a structure, but not limited thereto, to be directly accommodated in the washing and disinfecting bath 5.

### (Second Embodiment)

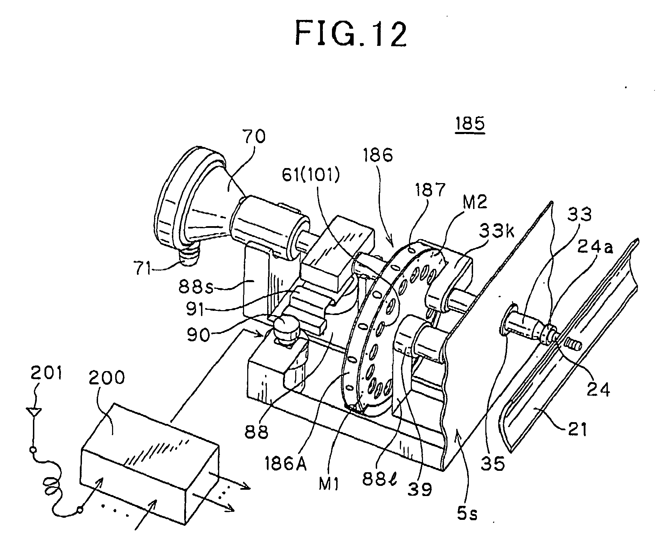

Reference is further made to FIGS. 12 to 15 to describe an endoscope washing and disinfecting apparatus of a second embodiment according to the present invention. Also, the same component parts of the present embodiment as those of the first embodiment bear like reference numerals to simply the description.

With the endoscope washing and disinfecting apparatus of the second embodiment, a feature resides in a structure of the brush unit 185 for the washing and a control to be performed for selectively using one of a plurality of brush chips 101 accommodated in the brush unit 185.

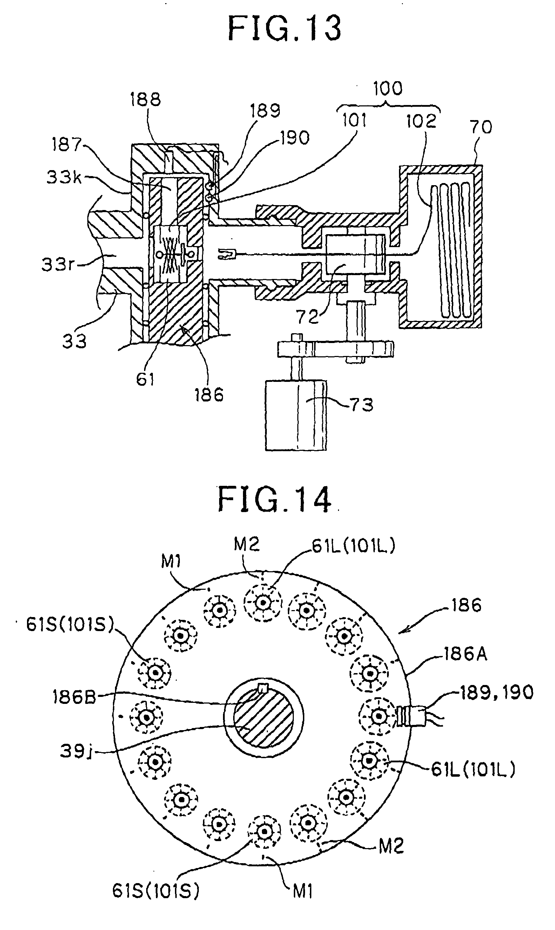

An external structure of the brush unit 185 is shown in FIG. 12 corresponding to FIG. 3 mentioned above. As shown in FIG. 12, the brush unit 185 includes a toric brush chip cartridge 186 with a certain thickness. The brush chip cartridge 186 has an outer periphery, formed with radially extending access windows 187, which is covered with a transparent plate for enabling each of the plurality of brush chip accommodating chambers 61 to be viewed from the outside via the outer periphery. Such an appearance is shown in FIG. 13.

The crank portion 33k carries thereon chip detecting sensors 188 that can be placed in face-to-face relation to the access windows 187, respectively. Each chip detecting sensor 188 generates output signals representing the presence of or the absence of the brush chip 101 in, for instance, optical or magnetic detection. This signal is applied to a control device 200 for processing to enable the presence of the brush chip 100 to be confirmed. The control device 200 is structured with a computer having a CPU like the control device 120 set forth above.

In addition, the brush chip cartridge 186 includes a toric casing 186A. The casing 186A has one sidewall on which two kinds of markers M1, M2 are provided in areas close proximity to each other for each brush chip accommodating chamber 61 for distinguishing a kind (that is, for instance, a kind of diameters) of the brush chip 101. Of these markers M1, M2, one marker M1 represents the brush chip 101 with a small diameter and the other marker M2 represents the brush chip 101L with a large diameter. The one marker M1 is marked with a single black circle "•" and the other marker M2 is marked with double black circles "••". In addition, the way of representing these markers may be selected in another way or may include magnetic markers using magnet or the like. In addition, the positions of the markers M1, M2, provided on the cartridge in a circumferential direction thereof, also represent circumferential positions of the accommodating chambers 61. That is, the markers M1, M2 double as a function of performing the positioning in the circumferential direction and a function of distinguishing the kind of the brush chips.

With the present embodiment, two kinds of brush chips 101S, 101L, having the brush portions 101h with small and large diameters, are prepared in a plurality of pieces. The brush chip accommodating chambers 61 may preferably include two kinds of accommodating chambers 615, 61L in various sizes such as small and large diameters. As one example, a total of sixteen brush chip accommodating chambers 61 is classified into eight pieces of brush chip accommodating chambers 61S with a small diameter and eight pieces of brush chip accommodating chambers 61L with a large diameter as shown in FIG. 13, which are charged with brush chips 101S (101L) with a small or large diameter depending on these kinds. In addition, the accommodating chambers 61S, 61L of two kinds are located in the cartridge 186 in a circumferential direction thereon at equidistantly spaced positions.

As shown in FIG. 13, further, the crank portion 33k is provided with two marker detecting sensors 189, 190 for the markers M1, M2, respectively, that can optically read out the markers M1, M2. These sensors 189, 190 generate output signals that are applied to the control device 200. The control device 200 discriminates which of the marker M1 "•" and the marker M2 "••" belongs to the detected marker. That is, a judgment is made on which of the brush chip 101S with the small diameter and the brush chip 101L with the large diameter belongs to the detected marker.

With the present embodiment, further, the control device 200 is associated with an antenna 201 mounted on the main body unit 3 to have capability of automatically reading out unique information (at least a size of a duct to be subjected to the brush washing) of the endoscope 20 accommodated in the washing and disinfecting bath 5. In performing such reading operation, non-contact remote communicating means, incorporated in the endoscope or mounted thereon at an external area thereof, may be employed as one example.

Therefore, the control device 200 can discriminate the size of the duct of the endoscope.

As shown in FIG. 14, further, the motor shaft 39j of the motor unit 39 has a convex-shaped ridge that can be aligned with a key recess of the brush chip cartridge 186. Such positioning alignment enables a circumferentially initial rotary position of the brush chip cartridge 186, that is, a circumferentially initial rotary position of the brush chip accommodating chamber 61 (61S or 61L) to be determined. The control device 200 is configured so as to preliminarily store such an initial rotary position for distinguishing the used brush chip 101 (101S or 101L) from an unused one based on a rotating distance relative to the initial rotary position and storing discriminated result. Therefore, the brush unit 185 has a function to prevent the used brush chips (101S or 101L) from being used again.

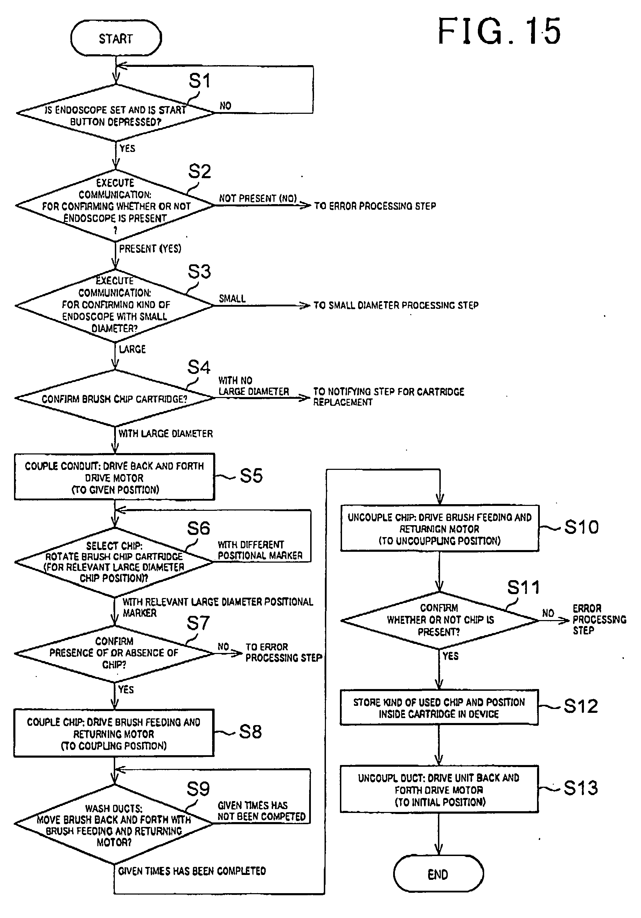

Now, an exemplary basic sequence of operations to be executed with the control device 200 will be described with reference to FIG. 15. First, a user places the endoscope 20 to be washed and disinfected on the tray 10 and sets the tray 10 on the washing and disinfecting bath 5, after which the top cover 4 is closed. Subsequently, the user operates the mode selection switch and the start switch provided on the operation panel 8.

As shown in FIG. 15, the control device 200 stands by for the mode switch and the start switch being operated (stepS1). In response to the operations of these switches, a sequence of operations is executed for confirming whether or not the endoscope is present in the washing and disinfecting bath 5 (step S2), what is a kind of the endoscope 20 (whether the duct has a large size or a small size: step S3) and whether or not the brush chip 101L or 101S with the determined large size or small size is present in the brush chip cartridge 186 (step S4).

Among these operations, the presence of and the kind of the endoscope 20 is confirmed upon reading out unique information of the endoscope 20 on communication. If no endoscope 20 is present in the washing and disinfecting bath 5 (step S2 with "NO"), the control device 200 proceeds the operation to an error processing step in which the operation panel 8 is provided with an error display. In contrast, under a circumstance where the endoscope is set in the washing and disinfecting bath 5 (step S2 with "YES"), the operation proceeds to step S4 in the presence of the endoscope 20 with the duct in the large diameter. In case of the small diameter, the operation proceeds to the same series of operational steps as those achieved in the case of the large diameter.

Further, a judgment in step S4 is executed using stored information based on the markers M1, M2 and the rotary position incremented from the initial position. If a judgment is made in step S4 that the brush chip 101L is present in the current brush chip cartridge 186, the operations subsequent to step S5 are executed. In contrast, if a judgment is made that only the brush chip 101S with the small diameter is present, a display is provided on the operational panel 8 for replacement of the cartridge.

Now, if the brush chip 101L with the large diameter is present, the control device 200 drives the back and forth drive motor 90 to cause the nozzle 33 to be coupled to the fitting 24a of the therapeutic-instrument inserting section 24 of the endoscope 20 (step S5). Then, for the brush chip 101L with the large diameter to be selected, the control device 200 rotates the motor unit 39 to the rotary position in which the relevant chip is present, upon which a judgment is made whether or not the rotary position is proper (step S6). If the judgment is made to be positive with "YES", the brush chip 101L with the large diameter intervenes in a linear communicating passage between the flow passage 33r of the nozzle 33 and the accommodating chamber 70s.

Under such a state, the control device 200 makes confirmation using the chip detecting sensor 188 as viewed from the access window 187 whether or not the brush chip 101L with such a large diameter is actually present (step S7). If it is turned out that the brush chip 101L is not actually present (step 57 with "NO"), a given error operation is executed. In contrast, if the brush chip 101L is present, the operation proceeds to step S8.

Therefore, the control device 200 drives the back-feed motor 73 until the shaft 102 arrives at a mounted position (step S8). This enables the brush chip 101L to be mounted (engaged) to the shaft 102. In addition, the back-feed motor 73 is driven a given number of times based on a basic sequence of washing operations, which are preliminarily determined, for advancing or retarding movements, thereby causing the brush chip 101L, located at the distal end of the shaft 102, to move back and forth in the therapeutic-instrument insertion duct 43 of the endoscope 20 (step S9). This allows the duct 43 to be washed.

With the washing completed in such away, the brush feeding-returning motor 73 is driven until the shaft 102 comes to an uncoupling position (step S10). This enables the brush chip 101L to be uncoupled from the shaft 102. Thereafter, the operation is executed to confirm the brush chip 101L in the same way as the step S7 (step S11). Therefore, even under a circumstance where the brush chip 101L is uncoupled from the shaft 102 during the washing or subsequent retracting movement thereof and no brush chip 101L is returned, such a situation can be immediately grasped.

In next step, the control device 200 stores the kind of the used brush chip 101L and the relevant position associated with the brush chip cartridge 186 for a subsequent washing and disinfecting work (step S12). In addition, the control device 200 drives the back and forth drive motor 90 to cause the nozzle 33 to be uncoupled from the fitting 24a of the therapeutic-instrument mounting section of the endoscope 20, thereby retracting the nozzle 33 to the initial position (step 513). By so doing, a series of operations are completed.

Accordingly, upon executing such a series of operations, the advantageous effect of the first embodiment can be obtained and, in addition thereto, the same brush chip cartridge 186 can address a task of washing a plurality of kinds of endoscope having ducts with plural types of diameters. This results in an increase in a general versatility of the cartridge.

Further, even under a circumstance where due to some failures, the endoscope and the middle of the associated duct are clogged with the brush chip 101, a judgment can be automatically made using the chip detecting sensor 188 to find whether or not the cartridge 186 is reliably returned and present. This makes it possible to make a proper judgment to address an issue of the brush chip 101 remaining in the duct.

With the embodiments set forth above, furthermore, all of the some kinds of brush chips 101S or 101L, charged in the brush chip cartridge 186, are used once. Under such a situation, even if the remaining some kinds of brush chips 101S or 101L remain unused, the brush chip cartridge 186 needs to be replaced with new one. However, though not shown, readable IC chips may be attached to the brush chip cartridge 186 such that the main body unit 3 can access and write positional information on used brush chips. By so doing, under a circumstance where any one of or both of the brush chips 101S or 101L of two kinds still remain in the duct, the brush chip cartridge 186 can be replaced with another brush chip cartridge depending on needs. That is, when the brush chip cartridge 186 is returned to the original position, the IC chips stores information on whether or not the brush chips are used and using such information enables unused brush chips to be selectively used. In addition, the remaining brush chips can be mounted on another cartridge for a subsequent use.

In addition, the brush chips of the plural kinds described above are not limited to the brush chips of the two kinds. More than three kinds of brush chips may be employed. Moreover, one cartridge may be charged with a plurality of brush chips (with brush portions with the same diameter) of one kind and a plurality of such cartridges may be prepared upon which each cartridge is creatively used to suit a diameter of a duct of an endoscope.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An endoscope washing and disinfecting apparatus used to, at least, wash a duct of the endoscope, comprising;
a washing brush having a brush chip used for the washing and a shaft having a distal end to which the brush chip is detachably loaded;
a nozzle having a flow passage and being detachably coupled to a connecting port of the duct of the endoscope;
a brush-chip accommodating unit having a brush-chip accommodating chamber communicable to the flow passage;
a shaft accommodating unit having a shaft accommodating chamber communicable to the brush-chip accommodating chamber, the shaft being selectively accommodated in the shaft accommodating chamber; and
a shaft driving unit driving the shaft to be fed to the duct of the endoscope from the shaft accommodating chamber and to be returned to the shaft accommodating chamber from the duct of the endoscope;
wherein the shaft driving unit comprises
coupling means for coupling a shaft engaging member disposed at a distal end of the shaft to a brush-chip engaging member of the brush chip when the shaft is fed into the brush-chip accommodating chamber, and
uncoupling means for uncoupling the shaft engaging member of the shaft from the brush-chip engaging member when the shaft is returned to the brush-chip accommodating chamber.

2. The apparatus of claim 1, wherein
the brush-chip accommodating chamber comprises an inward flange protruding toward an inside therein and having a communicating hole, the flange being located at least near the shaft accommodating chamber in the brush-chip accommodating chamber, and,
the brush chip comprises a stopper made to touch the inward flange during the retuning so that the brush chip is prevented from coming out from the brush-chip accommodating chamber to the shaft accommodating chamber via the communicating hole.

3. The apparatus of claim 2, wherein the brush-chip accommodating unit is rotatable to the nozzle,
the apparatus comprising
a rotation controlling unit controllably rotating the brush-chip accommodating unit.

4. The apparatus of claim 3, wherein
the brush-chip accommodating unit comprises the brush-chip accommodating chamber by a plurality of chambers, wherein the brush chip is adapted to be accommodated in each of the plural brush-chip accommodating chambers, and
the rotation controlling unit is configured to controllably rotate the brush-chip accommodating unit such that any one of the plural brush-chip accommodating chambers is made to communicate with both the flow passage of the nozzle and the shaft accommodating chamber.

5. The apparatus of claim 4, wherein
the shaft driving unit comprise means for returning the brush chip to the same brush chip chamber as that in which the brush chip is accommodated before coupling with the shaft.

6. The apparatus of claim 5, wherein
the rotation controlling unit is configured to rotate the brus h-chip accommodating unit after the returned brush chip has been accommodated such that any of brush chip chambers adjacent to the currently used brush chip chamber is made to communicate with both the flow passage of the nozzle and the shaft accommod ating chamber.

7. The apparatus of claim 6, wherein the shaft accommodating unit is detachably coupled to the nozzle.

8. The apparatus of claim 7, wherein the washing brush, the nozzle, the brush-chip accommodating unit, the shaft accommodating unit, and the shaft driving unit are incorporated in a single unit and the single unit is detachably loaded to the apparatus.

9. The apparatus of claim 1, wherein the brush-chip accommodating unit comprises
a casing in which the brush-chip accommodating chamber is formed to removably accommodate therein a brush chip to be detachably loaded to a distal end of a shaft, the brush chip being automatically driven to go back and forth in and along the duct of the endoscope, and
a flange portion formed in the brush-chip accommodating chamber and formed to allow the removable accommodation of the brush chip.

10. The apparatus of claim 9, wherein the brush-chip accommodating chamber is provided by a plurality of chambers and the brush chip is detachably accommodated in each of the plural brush-chip accommodating chambers.

11. The apparatus of claim 10, comprising
selection means for selecting a desired brush-chip accommodating chamber among the plural brush-chip accommodating chambers; and
derive means for driving the shaft driving unit so that the brush chip accommodated in the brush-chip accommodating chamber selected by the selecting means is for washing the duct.

12. A brush unit comprising;
a washing brush having a brush chip used for washing a duct of an endoscope and a shaft having a distal end to which the brush chip is detachably loaded;
a nozzle having a flow passage and being detachably coupled to a connecting port of the duct of the endoscope;
a brush-chip accommodating unit having a brush-chip accommodating chamber communicable to the flow passage;
a shaft accommodating unit having a shaft accommodating chamber communicable to the brush-chip accommodating chamber, the shaft being selectively accommodated in the shaft accommodating chamber; and
a shaft driving unit driving the shaft to be fed to the duct of the endoscope from the shaft accommodating chamber and to be returned to the shaft accommodating chamber from the duct of the endoscope;
wherein the shaft driving unit comprises
coupling means for coupling a shaft engaging member disposed at a distal end of the shaft to a brush-chip engaging member of the brush chip when the shaft is fed into the brush-chip accommodating chamber, and
uncoupling means for uncoupling the shaft engaging member of the shaft from the brush-chip engaging member when the shaft is returned to the brush-chip accommodating chamber.

13. The brush unit of claim 12, wherein
the brush-chip accommodating chamber comprises an inward flange protruding toward an inside therein and having a communicating hole, the flange being located at least near the shaft accommodating chamber in the brush-chip accommodating chamber, and,
the brush chip comprises a stopper made to touch the inward flange during the retuning so that the brush chip is prevented from coming out from the brush-chip accommodating chamber to the shaft accommodating chamber via the communicating hole.

14. The brush unit of claim 13, wherein the brush-chip accommodating unit is rotatable to the nozzle,
the brush unit comprising
a rotation controlling unit controllably rotating the brush-chip accommodating unit.

15. The brush unit of claim 14, wherein
the brush-chip accommodating unit comprises the brush-chip accommodating chamber by a plurality of chambers, wherein the brush chip is adapted to be accommodated in each of the plural brush-chip accommodating chambers, and
the rotation controlling unit is configured to controllably rotate the brush-chip accommodating unit such that any one of the plural brush-chip accommodating chambers is made to communicate with both the flow passage of the nozzle and the shaft accommodating chamber.

16. The brush unit of claim 15, wherein
the shaft driving unit comprise means for returning the brush chip to the same brush chip chamber as that in which the brush chip is accommodated before coupling with the shaft.

17. The brush unit of claim 16, wherein
the rotation controlling unit is configured to rotate the brus h-chip accommodating unit after the returned brush chip has been accommodated such that any of brush chip chambers adjacent to the currently used brush chip chamber is made to communicate with both the flow passage of the nozzle and the shaft accommod ating chamber.

18. The brush unit of claim 17, wherein the shaft accommodating unit is detachably coupled to the nozzle.

19. A cartridge comprising
a casing in which a brush-chip accommodating chamber is formed to removably accommodate therein a brush chip to detachably be loaded to a distal end of a shaft, the brush chip being driven go back and forth in and along a duct of an endoscope; and
a flange portion formed in the brush-chip accommodating chamber and formed to allow the removable accommodation of the brush chip.

20. The cartridge of claim 19, wherein the brush chip comprise a brush engaging member to be detachably engaged with a shaft engaging member mounted at the distal end of the shaft.

21. The cartridge of claim 20, wherein
the brush-chip accommodating chamber comprises an inward flange protruding toward an inside therein and having a communicating hole, and
the brush chip comprises a stopper made to touch the inward flange during the retuning so that the brush chip is prevented from coming out from the brush-chip accommodating chamber to the shaft accommodating chamber via the communicating hole.

22. The brush unit of claim 19, wherein
the brush-chip accommodating chamber is formed by a plurality of chambers and the brush chip is removably accommodated in at least one of the plural brush-chip accommodating chambers.

23. The brush unit of claim 22, wherein the plural brush-chip accommodating chambers consist of a plurality of types of brush-chip accommodating chambers which are different in sizes, type by type, depending on a plurality of types of bush chips which are different in sizes, type by type.

24. The brush unit of claim 23, wherein
the sizes of the plurality of types of brush chips are expressed by sizes of diameters of the brush chips and differences in the diameters of the plurality of types of brush chips correspond to differences in diameters of a plurality of types of ducts of endoscopes.

25. The brush unit of claim 22, wherein
the casing is formed into an annular type of casing and
the plural brush-chip accommodating chambers are formed in the annular casing in an arrangement where each chamber passes through the casing in an axial direction thereof and the chambers are mapped in a circumferential direction thereof.

26. The brush unit of claim 25, wherein
the casing is formed to have plural markings each indicating a position of each of the plural brush-chip accommodating chambers.

27. A brush unit comprising
a brush chip having a hair portion for brushing;
a shaft having a distal end to which the brush chip is loaded; and
coupling means for coupling the brush chip and the shaft to each other.

28. The brush unit of claim 27, wherein the coupling mea ns is configured to detachably couple the brush chip to the distal end of the shaft along an axial direction of the shaft.

29. The brush unit of claim 28, wherein, when the bush chip is loaded to the shaft, the hair portion is directed in a radial direction of the shaft perpendicular to the axial direction so as to apply the brushing to an inside of a duct of an endoscope.

30. An endoscope washing apparatus used to wash a duct of the endoscope, comprising;
a washing brush having a brush chip used for the washing and a shaft having a distal end to which the brush chip is detachably loaded;
a nozzle having a flow passage and being detachably coupled to a connecting port of the duct of the endoscope;
a brush-chip accommodating unit having a brush-chip accommodating chamber communicable to the flow passage;
a shaft accommodating unit having a shaft accommodating chamber communicable to the brush-chip accommodating chamber, the shaft being selectively accommodated in the shaft accommodating chamber; and
a shaft driving unit driving the shaft to be fed to the duct of the endoscope from the shaft accommodating chamber and to be returned to the shaft accommodating chamber from the duct of the endoscope;
wherein the shaft driving unit comprises
coupling means for coupling a shaft engaging member disposed at a distal end of the shaft to a brush-chip engaging member of the brush chip when the shaft is fed into the brush-chip accommodating chamber, and
uncoupling means for uncoupling the shaft engaging member of the shaft from the brush-chip engaging member when the shaft is returned to the brush-chip accommodating chamber.
